(19) Europäisches Patentamt
European Patent Office
Office européen des brevets

(11) Numéro de publication : **0 354 094 B1**

(12) # FASCICULE DE BREVET EUROPEEN

(45) Date de publication du fascicule du brevet : **27.12.91 Bulletin 91/52**

(51) Int. Cl.$^5$ : **C07D 209/34**, C07D 401/12, A61K 31/40, A61K 31/44, A61K 31/475

(21) Numéro de dépôt : **89402102.1**

(22) Date de dépôt : **25.07.89**

(54) **Dérivés d'indolone, leur préparation et leur application en thérapeutique.**

(30) Priorité : **03.08.88 FR 8810482**

(43) Date de publication de la demande : **07.02.90 Bulletin 90/06**

(45) Mention de la délivrance du brevet : **27.12.91 Bulletin 91/52**

(84) Etats contractants désignés : **AT BE CH DE ES FR GB GR IT LI LU NL SE**

(56) Documents cités :
**US-A- 3 732 248**
**JOURNAL OF MEDICINAL CHEMISTRY, vol. 7, no. 2, mars 1964, pages 135-141; R.G. TABORSKY et al.: "The synthesis and preliminary pharmacology of some 9H-pyrido[3,4-b]indoles (beta-carbolines) and tryptamines related to serotonin and melatonin"**

(73) Titulaire : **SYNTHELABO**
**58 rue de la Glacière**
**F-75013 Paris (FR)**

(72) Inventeur : **Manoury, Philippe**
**L'Orée de Verrières 38 Avenue des Vaupépins**
**F-91370 Verrières Le Buisson (FR)**
Inventeur : **Binet, Jean**
**12 Hameau de la Gondole**
**F-91650 Breuillet (FR)**
Inventeur : **Obitz, Daniel**
**91, rue Boucicaut**
**F-92250 Fontenay Aux Roses (FR)**
Inventeur : **Defosse, Gérard**
**29, rue de Tolbiac**
**F-75013 Paris (FR)**
Inventeur : **Dewitte, Elisabeth**
**38, Avenue D'Orgeval**
**F-95210 Saint-Gratien (FR)**
Inventeur : **Véronique, Corinne**
**5, rue Jacques Duclos Apt. 4**
**F-94800 Villejuif (FR)**

(74) Mandataire : **Thouret-Lemaitre, Elisabeth et al**
**SYNTHELABO Service Brevets 22, avenue Galilée**
**F-92352 LE PLESSIS ROBINSON CEDEX (FR)**

EP 0 354 094 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet des dérivés d'indolone, leur préparation et leur application en thérapeutique.

Les composés de la présente invention répondent à la formule générale (I) donnée en annexes, dans laquelle

— $R_1$ est un atome d'hydrogène ou d'halogène ou un radical $(C_{1-4})$alkyle,

— $R_2$ est un atome d'hydrogène ou un radical $(C_{1-4})$alkyle,

— $R_3$ est un atome d'hydrogène, un radical $(C_{1-4})$alkyle ou un radical S-$(C_{1-4})$alkyle,

— $R_4$ est un radical phényle, chloro-phényle, naphtalényle-1, méthoxy-7 naphtalényle-1, méthoxy-6 indanyle-1, méthoxy-2 pyridinyle-6, méthoxy-3 pyridinyle-2, isoquinoléinyle, méthoxy-7 isoquinoléinyle-1, méthoxy-7 tétrahydro-1,2,3,4 naphtalényle-1 ou fluoro-7 naphtalényle-1.

Les composés présentent un ou plusieurs atomes de carbone asymétriques.

Les diastéréoisomères et les énantiomères de ces composés font partie de l'invention.

Les composés de l'invention peuvent être préparés selon les schémas réactionnels donnés en annexes 1 et 2. Lorsque dans les composés (I), $R_3$ est H, on prépare les composés selon le schéma réactionnel 1 : on fait réagir un composé (II), $R_1$-4 benzènamine, avec de l'hypochlorite de tertiobutyle, puis avec de l'éthylthio-3 γ-butyrolactone (A.F. Wagner, US 2,842,590, CA 5218220 d) et enfin avec de la triéthylamine, ce qui conduit au composé (III) que l'on transforme en tosylate que l'on fait réagir avec une $R_4$-1 pipérazine (IV) ; on obtient alors un composé (V)

— que l'on désulfure directement pour obtenir les composés (I) dans lesquels $R_2$ est H,

— que l'on fait réagir en présence d'hydrure de sodium avec un composé $R_2X$ (dans lequel X est un atome d'halogène) puis on désulfure le composé intermédiaire, pour obtenir les composés (I) dans lesquels $R_2$ est un radical $(C_{1-4})$alkyle.

Lorsque dans les composés (I) $R_3$ est différent de H, on prépare les composés selon le schéma réactionnel 2 : On fait réagir une ($R_1$-4) benzènamine (II) avec de l'hypochlorite de tertiobutyle, puis avec l'éthylthio-2 $R_3$-2 éthanoate d'éthyle puis avec de la triéthylamine, ce qui conduit au composé (VI) que l'on fait réagir en présence d'hydrure de sodium avec un composé $R_2X$ puis on désulfure le composé intermédiaire en composé (VII) que l'on fait réagir avec un composé (VIII) pour obtenir les composés (I).

Les composés (II) sont décrits dans la littérature. Les composés (IV) sont décrits par la Demanderesse dans le brevet français n° 88.10481 ou dans la demande de brevet européen n° 89401330.9 ou dans la littérature. Les composés (VIII) sont préparés à partir des composés (IV) selon les méthodes classiques décrites dans la littérature.

Les composés de l'invention comportant un carbone asymétrique dans le radical

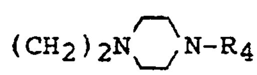

peuvent exister sous la forme d'énantiomères qui peuvent être préparés par synthèse stéréospécifique ; c'est-à-dire par réaction entre un tosylate du composé de formule (III) et un énantiomère de la pipérazine de formule (IV).

Les exemples 5 et 6 illustrent cette préparation.

Les exemples suivants illustrent l'invention. Les analyses et les spectres IR et RMN confirment la structure des composés.

EXEMPLE 1

Fumarate de fluoro-5 [[(méthoxy-7 tétrahydro-1,2,3,4 naphtalényl-1)-4 pipérazinyl-1]-2 éthyl]-3 dihydro-1,3 2H indolone-2.

1.1 (Ethylthio)-3 fluoro-5 (hydroxy-2 éthyl)-3 dihydro-1,3 2H-indolone-2.

On refroidit à −65°C une solution de 22,2 g (0,2 mole) de fluoro-4 aniline dans 650 ml de chlorure de méthylène et ajoute alors goutte à goutte 21,4 ml d'hypochlorite de t-butyle en solution dans 90 ml de $CH_2Cl_2$. On agite 1/4 d'heure après la fin de l'addition et ajoute en 1 heure environ, 26,2 g (0,2 mole) d'(éthylthio)-3 γ-bulyroiactone dilués dans 90 ml de $CH_2Cl_2$.

On maintient, sous agitation à −65°C, pendant 2 heures et ajoute 27,5 ml de triéthylamine dilués dans 90 ml de $CH_2Cl_2$. On laisse la température du mélange revenir à 20°C, laisse une nuit au repos. On verse le

mélange réactionnel dans de l'eau, décante, sèche la phase organique, filtre, évapore. On purifie le solide résultant par chromatographie sur silice (éluant acétate d'éthyle).

On obtient l'(éthylthio)-3 fluoro-5 (hydroxy-2 éthyl)-3 dihydro-1,3 2H indolone-2.

F = 125°C.

1.2 Tosylate de (éthylthio)-3 fluoro-5 (hydroxy-2 éthyl)-3 dihydro-1,3 2H-indolone-2.

On refroidit à 5°C environ une solution de 19 g (0,075 mole) d'(éthylthio)-3 fluoro-5 (hydroxy-2 éthyl)-3 dihydro-1,3 2H-indolone-2 dans 150 ml de pyridine.

On ajoute alors par portions 15,6 g (0,082 mole) de chlorure de méthyl-4 benzènesulfonyle.

On laisse la température du mélange revenir à 20°C et on laisse le mélange réactionnel la nuit au repos. On verse le mélange dans de l'eau, acidifie, extrait avec $CH_2Cl_2$. On lave la phase organique avec de l'eau, sèche, filtre, évapore. On obtient le tosylate d'(éthylthio)-3 fluoro-5 (hydroxy-2 éthyl)-3 dihydro-1,3 2H-indolone-2.

F = 105°C.

1.3 (Ethylthio)-3 fluoro-5 [[(méthoxy-7 tétrahydro-1,2,3,4 naphtalényl-1)-4 pipérazinyl-1]-2 éthyl]-3 dihydro-1,3 2H-indolone-2.

On chauffe à 110°C pendant 1 heure un mélange de 8,1 g (0,0328 mole) de (méthoxy-7 tétrahydro-1,2,3,4 naphtalényl-1)-1 pipérazine et 6,7 g (0,0164 mole) de tosylate d'(éthylthio)-3 fluoro-5 (hydroxy-2 éthyl)-3 dihydro-1,3 2H-indolone-2.

On refroidit le mélange et le purifie par chromatographie sur silice (éluant acétate d'éthyle/$CH_2Cl_2$ 50/50). On obtient une huile que l'on utilise brute pour la suite de la synthèse.

1.4 Fumarate de fluoro-5[[(méthoxy-7 tétrahydro-1,2,3,4 naphtalényl-1)-4 pipérazinyl-1]-2 éthyl]-3 dihydro-1,3 2H-indolone-2.

On porte au reflux pendant 2 heures 4,7 g (0,01 mole) du dérivé précédent en solution dans 100 ml d'éthanol en présence de 30 g de nickel de Raney désactivé.

On filtre le nickel, le rince avec de l'éthanol puis évapore le filtrat.

On prépare le fumarate de fluoro-5 [[(méthoxy-7 tétrahydro-1,2,3,4 naphtalényl-1)-4 pipérazinyl-1]-2 éthyl]-3 dihydro-1,3 2H-indolone-2 dans un mélange éthanol/éther.

F = 136°C.

EXEMPLE 2

Fumarate de fluoro-5 méthyl-1 [[(naphtalènyl-1)-4 pipérazinyl-1)-2 éthyl]-3 dihydro-1,3 2H-indolone-2.

2.1 Ethylthio-3 fluoro-5 [[(naphtalènyl-1)-4 pipérazinyl-1]-2 éthyl]-3 dihydro-1,3 2H-indolone-2.

Le produit est obtenu en suivant la technique décrite sous 1.3 à partir de 31,6 g (0,148 mole) de naphtalényl-1 pipérazine, 30,5 g (0,074 mole) de tosylate d'(éthylthio)-3 fluoro-5 (hydroxy-2 éthyl)-3 dihydro-1,3 2H-indolone-2.

F = 174-175°C.

2.2 Ethylthio-3 fluoro-5 méthyl-1 [[(naphtalènyl-1)-4 pipérazinyl-1]-2 éthyl]-3 dihydro-1,3 2H-indolone-2.

On ajoute 3,2 g (0,005 mole) du composé précédent en solution dans 25 ml de DMF, à une suspension de 0,25 g (0,0055 mole) de NaH à 50% dans 25 ml de DMF.

Lorsque le dégagement de l'hydrogène a cessé on introduit 0,5 ml d'iodure de méthyle puis chauffe le mélange à 50°C pendant 2 heures.

On verse le mélange dans de l'eau, extrait avec de l'éther.

Après traitement habituel on recueille le [[(naphtalènyl-1)-4 pipérazinyl]-2 éthyl]-3 éthylthio-3 fluoro-5 méthyl-1 dihydro-1,3 2H-indolone-2 sous forme d'huile que l'on désulfure en utilisant la technique décrite sous 1.4.

On obtient le fumarate de fluoro-5 méthyl-1 [(naphtalènyl-1)-4 pipérazinyl-1]-2 éthyle-3 dihydro-1,3 2H-indolone-2.

F = 198-200°C.

EXEMPLE 3

Fluoro-5 diméthyl-1,3 [[(naphtalènyl-1)-4 pipérazinyl-1]-2 éthyl]-3 dihydro 1,3 2H-indolone-2.

3.1 (Ethylthio)-3 fluoro-5 méthyl-3 dihydro-1,3 2H-indolone-2.

On prépare le composé comme décrit sous 1.1 à partir de 24,4 g (0,22 mole) de fluoro-4 aniline, 26,2 ml d'hypochlorite de t-butyle et 35,7 g (0,22 mole) d'(éthylthio)-2 propanoate d'éthyle.
F = 113-114°C.

3.2 (Ethylthio)-3 fluoro-5 diméthyl-1,3 dihydro-1,3 2H-indolone-2.

On méthyle 11,3 g (0,05 mole) d'(éthylthio)-3 fluoro-5 méthyl-3 dihydro-1,3 2H-indolone-2 comme décrit sous 2.2 à partir de 2,6 g (0,055 mole) de NaH à 50% et 3,7 ml d'iodure de méthyle.
F = 63-65°C.

3.3 Fluoro-5 diméthyl-1,3 dihydro-1,3 2H-indolone-2.

On désulfure 20,5 g du composé précédent dans 150 ml d'éthanol au reflux en présence de 120 g de nickel de Raney désactivé.
On obtient le composé sous forme d'huile.

3.4 Fluoro-5 diméthyl-1,3 [[(naphtalényl-1)-4 pipérazinyl-1]-2 éthyl]-3 dihydro-1,3 2H-indolone-2.

On ajoute 1,6 g (0,009 mole) du composé précédent en solution dans 10 ml de DMF à une suspension de 0,5 g (0,01 mole) de NaH à 50% dans 20 ml de DMF. Lorsque la sodation est achevée on ajoute 2,4 g (0,009 mole) de (chloro-2 éthyl)-1 (naphtalényl-1)-4 pipérazine diluée dans 10 ml de DMF puis chauffe le mélange à 60°C pendant 12 heures.
On verse le mélange dans de l'eau, extrait avec de l'éther, sèche, filtre, évapore.
Le produit est purifié par chromatographie sur colonne de silice. On obtient la fluoro-5 diméthyl-1,3 [(naphtalényl-1)-4 pipérazinyl-1]-2 éthyl]-3 dihydro-1,3 2H-indolone-2.
F = 144-146°C.

EXEMPLE 4

Fluoro-5 [[(méthoxy-7 naphtalényl-1)-4 pipérazinyl-1]-2 éthyl]-3 dihydro-1,3 2H-indolone-2.

4.1 (Ethylthio)-3 fluoro-5 [[(méthoxy-7 naphtalényl-1)-4 pipérazinyl-1]-2 éthyl]-3 dihydro-1,3 2H-indolone-2.

On chauffe à 130° pendant 45 minutes un mélange intime de 3,66 g (0,0151 mole) de (méthoxy-7 naphtalènyl-1)-1 pipérazine et de 3,09 g (0,00755 mole) de tosylate d'(éthylthio)-3 fluoro-5 (hydroxy-2 éthyl)-3 dihydro-1,3 2H-indolone-2.
On agite ensuite le milieu réactionnel entre l'eau alcaline et le chlorure de méthylène, décante la phase organique puis la lave et la sèche sur sulfate de magnésium.
Après évaporation du solvant l'huile résiduelle est éluée par le mélange chlorure de méthylène/acétone (90/10) sur une colonne de 250 g de silice.
Après concentration des fractions pures on obtient 2,8 g d'un solide blanc que l'on triture dans l'éther puis essore, lave et sèche. On obtient finalement 2,3 g d'un solide blanc dont le point de fusion est 180-2°C.

4.2 Fluoro-5 [[(méthoxy-7 naphtalènyl-1)-4 pipérazinyl-1]-2 éthyl]-3 dihydro-1,3 2H-indolone-2.

On chauffe au reflux pendant 3 heures une suspension de 5 g du solide précédent et 50 g de nickel de Raney (préalablement désactivé par l'acétone) dans 350 ml d'éthanol. Après filtration du nickel et évaporation du solvant le solide restant est dissous partiellement dans 2,2 l d'éther. Un insoluble est filtré et le filtrat est concentré à environ 60 ml puis laissé au repos au réfrigérateur.
Après plusieurs heures le solide cristallisé est essoré, lavé à l'éther et séché. On obtient 3,27 g d'un solide

blanc de point de fusion de 177-8°C.

EXEMPLE 5

Fumarate de fluoro-5[[(méthoxy-7 tétrahydro-1,2,3,4 naphtalényl-1)-4 pipérazinyl-1]-2 éthyl]-3 dihydro-1,3 2H-indolone-2 (isomère dextrogyre).

5.1. (Ethylthio)-3 fluoro-5 [[(méthoxy-7 tétrahydro-1,2,3,4 naphtalényl-1)-4 pipérazinyl-1]-2 éthyl]-3 dihydro-1,3 2H-indolone-2 (isomère dextrogyre).

On porte à la température du reflux, pendant 2 h, un mélange de 19,8 g (0,0803 mole) de l'isomère dextrogyre de la (méthoxy-7 tétrahydro-1,2,3,4 naphtalényl-1)-1 pipérazine et de 16,3 g (0,0398 mole) de tosylate de (éthylthio-3 fluoro-5 (hydroxy-2 éthyl)-3 dihydro-1,3 2H-indolone-2 dans 140 ml de toluène.

On agite le mélange en présence d'éther et d'hydroxyde de sodium 2N. Après décantation, on lave le mélange à l'eau, le sèche et évapore.

Après chromatographie sur colonne de silice en utilisant comme éluant un mélange $CH_2Cl_2$/méthanol de 98/2 à 98/4, on récupère 16,2 g de produit pur.

5.2. Fluoro-5 [[(méthoxy-7 tétrahydro-1,2,3,4 naphtalényl-1)-4 pipérazinyl-1]-2 éthyl]-3 dihydro-1,3 2H-indolone-2 (isomère dextrogyre).

On porte à la température du reflux pendant 2 h, 106 g de Nickel de Raney dans 700 ml d'acétone, puis on lave le mélange à l'alcool.

On ajoute alors 16 g (0,033 mole) du composé obtenu précédemment en solution dans 400 ml d'éthanol. On porte le mélange à la température du reflux pendant 2 h. On filtre le nickel, le rince avec de l'éthanol et évapore le filtrat. Le composé sous forme de base fond à 114°C.

On prépare le fumarate du composé obtenu dans un mélange d'éthanol et d'éther. F = 132°C.

$[\alpha]_D^{20}$ = +78,5° c = 1,04 méthanol

EXEMPLE 6

Fumarate de fluoro-5 [[(méthoxy-7 tétrahydro-1,2,3,4 naphtalényl-1)-4 pipérazinyl-1]-2 éthyl]-3 dihydro-1,3 2H-indolone-2 (isomère lévogyre).

6.1. (Ethylthio)-3 fluoro-5 [[(méthoxy-7 tétrahydro-1,2,3,4 naphtalényl-1)-4 pipérazinyl-1]-2 éthyl]-3 dihydro-1,3 2H-indolone-2 (isomère lévogyre).

On porte à 100°C, pendant 9 h, un mélange de 13,9 g (0,0564 mole) de l'isomère levogyre de la (méthoxy-7 tétrahydro-1,2,3,4 naphtalényl-1)-1 pipérazine, 20,06 g (0,0490 mole) de tosylate de (éthylthio)-3 fluoro-5 (hydroxy-2 éthyl)-3 dihydro-1,3 2H-indolone-2 et 9,4 g (0,112 mole) de carbonate acide de sodium dans 155 ml de toluène.

On filtre les sels minéraux puis évapore le filtrat. Après chromatographie sur colonne de silice avec comme éluant un mélange $CH_2Cl_2$/méthanol 97/3, on obtient 19,4 g de produit pur.

$[\alpha]_D^{20}$ = −81,5°.

6.2. Fluoro-5 [[(méthoxy-7 tétrahydro-1,2,3,4 naphtalényl-1)-4 pipérazinyl-1]-2 éthyl]-3 dihydro-1,3 2H-indolone-2 (isomère lévogyre).

On porte à ébullition, pendant 2 h, 127 g de nickel de Raney dans 850 ml d'acétone. On décante l'acétone et lave deux fois à l'éthanol.

On ajoute à ce mélange une solution de 19,3 g (0,04 mole) du composé obtenu prédécemment dans 480 ml d'éthanol absolu. On porte le mélange à la température du reflux pendant 1 h tout en agitant fortement.

On filtre le nickel de Raney, on évapore sous vide et reprend le mélange à l'éther. On filtre l'insoluble. Après évaporation, on obtient le produit sous forme de base.

On prépare le fumarate de ce composé dans un mélange éthanol/éther.

Après recristallisation dans du propanol, le produit fond à 132°C.

$[\alpha]_D^{20}$ = −78,3° c = 1,04 méthanol

Les composés de l'invention préparés à titre d'exemples sont représentés dans le tableau suivant.

Tableau

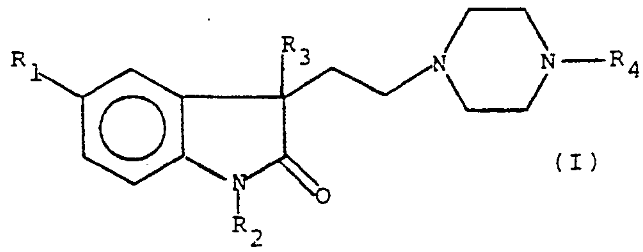

(I)

| Composé | $R_1$ | $R_2$ | $R_3$ | $R_4$ | F(°C) |
|---|---|---|---|---|---|
| 1 | H | H | $SC_2H_5$ | 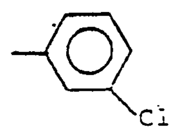 | 116-118 |
| 2 | H | H | H | 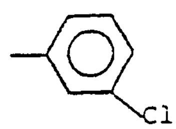 | 173-174 |
| 3 | H | H | $SC_2H_5$ | 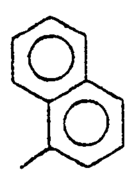 | 188-192 |
| 4 | H | H | H | 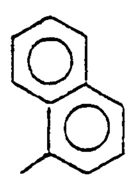 | 192-194 |
| 5 | H | H | H | 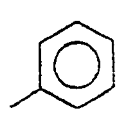 | 146-148 |

| Composé | $R_1$ | $R_2$ | $R_3$ | $R_4$ | F(°C) |
|---|---|---|---|---|---|
| 6 | F | H | $SC_2H_5$ | 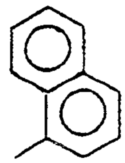 | 174-175 |
| 7 | F | H | H | 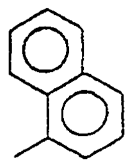 | 180-183 |
| 8 | F | H | $SC_2H_5$ | 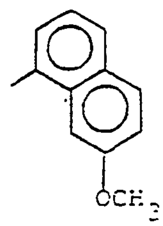 | 180-182 |
| 9 | F | H | H | 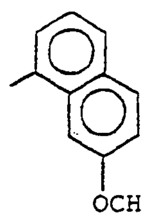 | 176-178 |
| 10 | F | H | H | 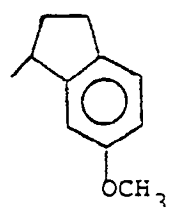 | 177-178 (fumarate) |
| 11 | F | H | H | 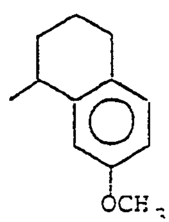 | 136 (fumarate) |
| 12 | Cl | H | $SC_2H_5$ | 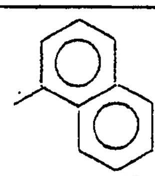 | 193-195 |

| Composé | $R_1$ | $R_2$ | $R_3$ | $R_4$ | F(°C) |
|---|---|---|---|---|---|
| 13 | Cl | H | H | 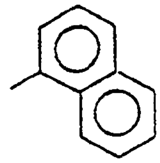 | 213-218 |
| 14 | $CH_3$ | H | $SC_2H_5$ | 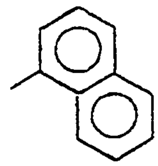 | 169-171 |
| 15 | F | $CH_3$ | H | 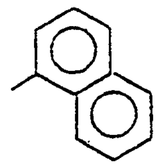 | 198-200 (fumarate) |
| 16 | F | $CH_3$ | $CH_3$ | 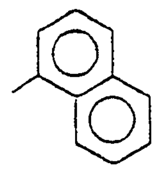 | 144-146 |
| 17 | F | H | H | 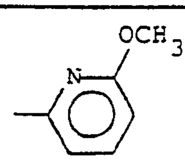 | 120 |
| 18 | F | H | H | 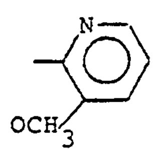 | 210 (fumarate) |
| 19 | F | H | H | 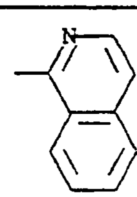 | 214-216 (fumarate) |
| 20 | F | $CH_3$ | H | 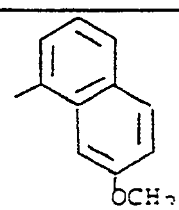 | 179-181 (fumarate) |

| Composé | $R_1$ | $R_2$ | $R_3$ | $R_4$ | F(°C) |
|---|---|---|---|---|---|
| 21 | F | H | H | 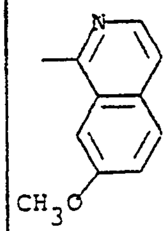 | 222-224 (fumarate) |
| 22 | F | $CH_3$ | H | 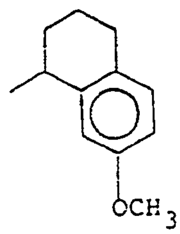 | 166-168 (maléate) |
| 23 | F | H | H | 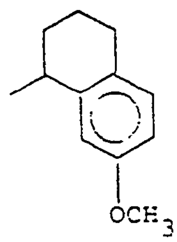 | isomère dextrogyre 132 fumarate |
| 24 | F | H | H | 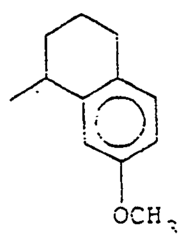 | isomère lévogyre 132 fumarate |
| 25 | F | H | S $C_2H_5$ | 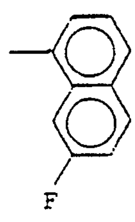 | 201-202 |
| 26 * | F | H | H | 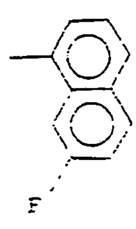 | 187-189 |

* Trace d'impureté (RMN).

Les composés de l'invention ont été soumis à une série d'essais pharmacologiques qui ont mis en évidence

leur intérêt comme substances à activités thérapeutiques.

Ainsi ils ont fait l'objet d'une étude quant à leur affinité pour les récepteurs sérotoninergiques du type 5-$HT_{1A}$. Les composés déplacent dans l'hippocampe du rat un ligand spécifique marqué, la [$^3$H]-hydroxy-8 dipropylamino-2 tétraline (désignée ci-après par "[$^3$H]-8-OH-DPAT") décrite par Gozlan et coll., Nature, (1983), 305, 140-142.

Les animaux utilisés sont des rats mâles Sprague-Dawley de 160 à 200 g. Après décapitation on en prélève le cerveau et on excise l'hippocampe. On broie le tissu dans un appareil Ultra-Turrax Polytron pendant 30 s à la moitié de la vitesse maximale dans 10 volumes de tampon Tris 50 mM d'un pH ajusté à 7,4 avec de l'acide chlorhydrique (soit 100 mg de tissu frais par ml). On lave les tissus homogénéisés trois fois à 4°C, en les centrifugeant à chaque fois à 48000×g et en remettant le culot en suspension pendant 10 mn dans du tampon frais refroidi. Finalement on met le dernier culot en suspension dans le tampon pour arriver à une concentration de 100 mg de tissu de départ par ml de tampon à 50 mM. On laisse ensuite incuber à 37°C pendant 10 mn. La liaison avec la [$^3$H]-8-OH-DPAT est déterminée par incubation de 10 µl de suspension de membranes dans un volume final de 1 ml de tampon contenant 10 µM de pargylline. Après l'incubation on récupère les membranes par filtration sur filtres Whatman GF/B qu'on lave trois fois avec des quantités aliquotes de 5 ml de tampon glacé. On extrait les filtres dans le liquide de scintillation et on en mesure la radioactivité par scintigraphie liquide. La liaison spécifique de la [$^3$H]-8-OH-DPAT est définie comme la quantité radioactive retenue sur les filtres et pouvant être inhibée par co-incubation dans la hydroxy-5 tryptamine à 10 µM. A une concentration de 1 nM de [$^3$H]-8-OH-DPAT la liaison spécifique représente de 70 à 80% de la radioactivité totale recupérée sur le filtre.

Pour chaque concentration de composés étudiés on détermine le pourcentage d'inhibition de la liaison avec la [$^3$H]-8-OH-DPAT, puis la concentration $CI_{50}$, concentration qui inhibe 50% de la liaison.

Pour les composés de l'invention les $CI_{50}$ se situent entre 0,001 et 0,3 µM.

L'activité centrale des composés de l'invention a été évaluée par leurs effets sur les "pointes PGO" (ponto-géniculo-occipitales) induites par la réserpine (test PGO-R) chez le chat, selon la méthode décrite par H. Depoortere, Sleep 1976, 3rd Europ. Congr. Sleep Res., Montpellier 1976, 358-361 (Karger, Basel 1977).

On administre des doses cumulatives de composés à étudier (de 0,01 à 3 mg/kg par voie intraveineuse) à des intervalles de temps de 30 mn, 4 h après injection intrapéritonéale d'une dose de 0,75 mg/kg de réserpine, à des chats curarisés, sous ventilation artificielle. On recueille les activités électroencéphalographique et phasique (pointes PGO-R) à l'aide d'électrodes corticales et profondes (genouillé latéral). Pour chaque dose de composé étudié on détermine le pourcentage de diminution du nombre de pointes PGO, puis la $DA_{50}$, dose active qui diminue de 50% ce nombre de pointes. Pour les composés de l'invention les $DA_{50}$ se situent entre 0,001 et 1 mg/kg par la voie intraveineuse.

Les résultats des essais montrent que certains des composés de formule générale (I) possèdent une grande affinité et une grande sélectivité pour les récepteurs sérotoninergiques de type 5-$HT_{1A}$. In vivo ils montrent une activité soit agoniste, soit agoniste partielle, soit antagoniste au niveau de ces récepteurs.

Certains composés de l'invention possèdent en outre une activité antisérotonine au niveau des récepteurs de type 5HT2.

Cette activité a été mise en évidence "in vitro" par déplacement de ligands fixés spécifiquement sur les récepteurs sérotoninergiques (test de binding SBS) et "in vivo" par antagonisme des effets de la sérotonine au niveau périphérique (test OES) et au niveau central (test AHT).

Test SBS : les composés de l'invention ont fait l'objet d'un essai de déplacement de la liaison (binding) du spiropéridol sur les récepteurs sérotoninergiques (5-HT2) du cortex cérébral du rat.

Pour cet essai on prélève les cerveaux de rats, on en dissèque le cortex et on l'homogénéise à 0°C dans 10 volumes d'un mélange contenant, par litre, 50 millimoles de tampon Tris/HCl à pH = 7,4, 120 millimoles de chlorure de sodium et 5 millimoles de chlorure de potassium. On centrifuge le mélange homogène à 40000×g pendant 10 mn puis, à deux reprises, on récupère le culot, on le lave en le mettant en suspension dans le même mélange tampon, on l'homogénéise de nouveau et on le centrifuge. Pour terminer on dilue le culot final dans le même mélange tampon à raison de 100 mg de tissu humide pour 1 ml de tampon.

On soumet alors le tissu à une incubation préalable de 10 mn à 37°C en présence de 10 micromoles/l de pargyline, puis à une incubation de 20 mn à 37°C en présence de $^3$H-spiropéridol (activité spécifique : 25,6 Ci par millimole) à la concentration de 0,3 nanomole/l et de composé à étudier à des concentrations allant de 0,0001 à 100 micromoles/l.

On prélève des aliquots de 1 ml que l'on filtre sous vide, on lave les filtres deux fois avec 5 ml de tampon froid et on les sèche. La radioactivité est mesurée dans le toluène en présence de 5 g/l de diphényl-2,5 oxazole (PPO) et 0,1 g/l de bis-(phényl-5 oxazolyl-2)-1,4 benzène (POPOP).

Pour évaluer l'activité des composés on établit la courbe du pourcentage d'inhibition de la liaison spécifique de $^3$H-spiropéridol en fonction de la concentration en drogue déplaçante. On détermine graphiquement la

concentration $IC_{50}$, concentration qui inhibe 50% de la liaison spécifique. La liaison spécifique est définie comme étant la liaison déplacée par 100 micromoles/l de 5-HT.

Les concentrations $IC_{50}$ des composés de l'invention se situent pour la plupart entre 1 et 50 nanomoles/l.

Test OES : l'activité antisérotoninergique des composés de l'invention a également été mise en évidence par leur effet sur l'oedème provoqué chez le rat par la sérotonine, selon la méthode décrite par Maling et coll., J. Pharmacol. Exp. Therap., 191 (2), 300-310 (1974).

Les animaux sont des rats mâles de souche CD (Ch. River, France) pesant 120 à 150 g, à jeun depuis 18 h et répartis en blocs randomisés.

Les composés, mis en solution ou en suspension dans du Tween $80^R$ à 1%, sont administrés par voie orale à raison de 0,5 ml pour 100 g de poids corporel, 1 h avant l'injection sous-plantaire, dans l'une des pattes postérieures, de 1 µg de sérotonine (en solution dans du sérum physiologique stérile, sous un volume de 0,1 ml). Le volume de l'oedème est mesuré 1 h après l'injection de sérotonine au moyen d'un pléthysmomètre à mercure Ugo Basile. La $DA_{40}$ (dose qui diminue de 40% le volume de l'oedème, par rapport aux animaux témoins) est déterminée graphiquement.

La $DA_{40}$ des composés de l'invention, déterminée par voie orale est comprise entre 0,1 et 2 mg/kg.

Test AHT : l'activité antisérotoninergique des composés a été étudiée quant à leur effet sur l'antagonisme des "head--twitches" (ébrouements de la tête) provoqués par le L-5-hydroxy-tryptophane (L-5-HTP) chez la souris, selon la méthode décrite par Corne et coll., Br. J. Pharmacol., 20, 106-120 (1962).

Les souris (mâles CD1, Charles River France ; 18-22 g de poids corporel) reçoivent les produits à étudier, à doses croissantes, ou le solvant, par voie intrapéritonéale ou orale, simultanément (voie i.p.) ou soixante minutes avant (voie orale) une injection de L-5-HTP à la dose de 250 mg/kg par voie sous-cutanée. Quarante-cinq minutes après cette injection de 5-HTP, le nombre d'ébrouements est compté, pour chaque souris, pendant une minute.

Pour chaque traitement, la moyenne des ébrouements, ainsi que le pourcentage de variation par rapport au lot témoin, sont calculés.

A partir de la courbe effet-dose, on détermine la $DA_{50}$ (dose active 50% ou dose qui diminue de 50% le nombre moyen d'ébrouements par rapport aux animaux témoins) par la méthode graphique de Miller et Tainter (Proc. Soc. Exp. Biol. Med., (1944), 57, 261).

Les $DA_{50}$ des composés de l'invention se situent entre 0,05 et 2 mg/kg par voie intrapéritonéale et entre 0,1 et 4 mg/kg par voie orale.

Les composés de l'invention peuvent être utiles pour le traitement de la migraine, l'anxiété, la dépression, l'obésité, la schizophrénie, les spasmes vasculaires ou gastrointestinaux, l'hypertension et l'agrégation plaquettaire, et comme antiémétiques.

Les composés de l'invention peuvent être administrés par voie orale ou parentérale, en association avec tout excipient approprié.

La posologie quotidienne peut aller de 1 à 1000 mg.

SCHEMA 1

ANNEXE 1

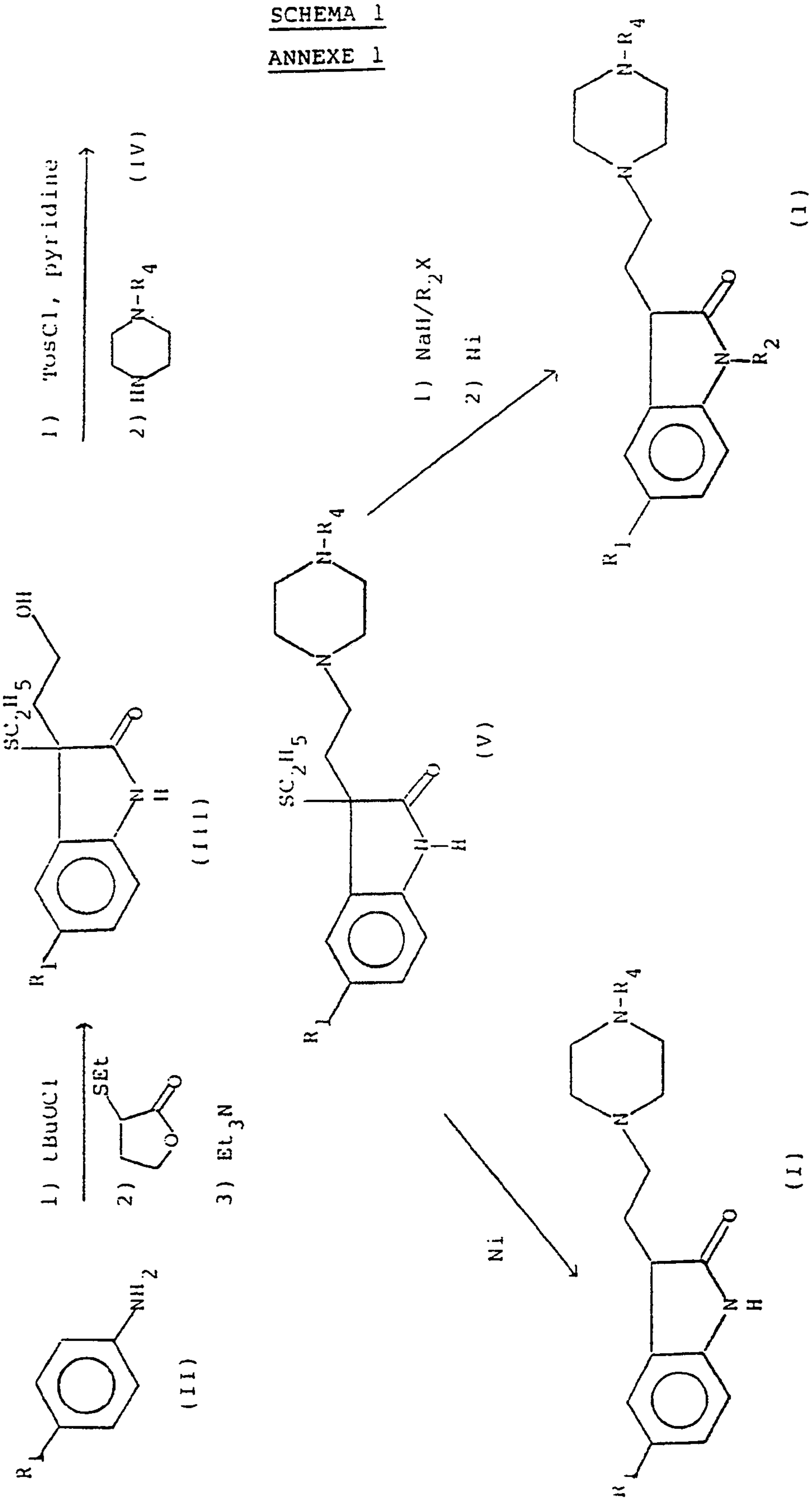

SCHEMA 2

ANNEXE 2

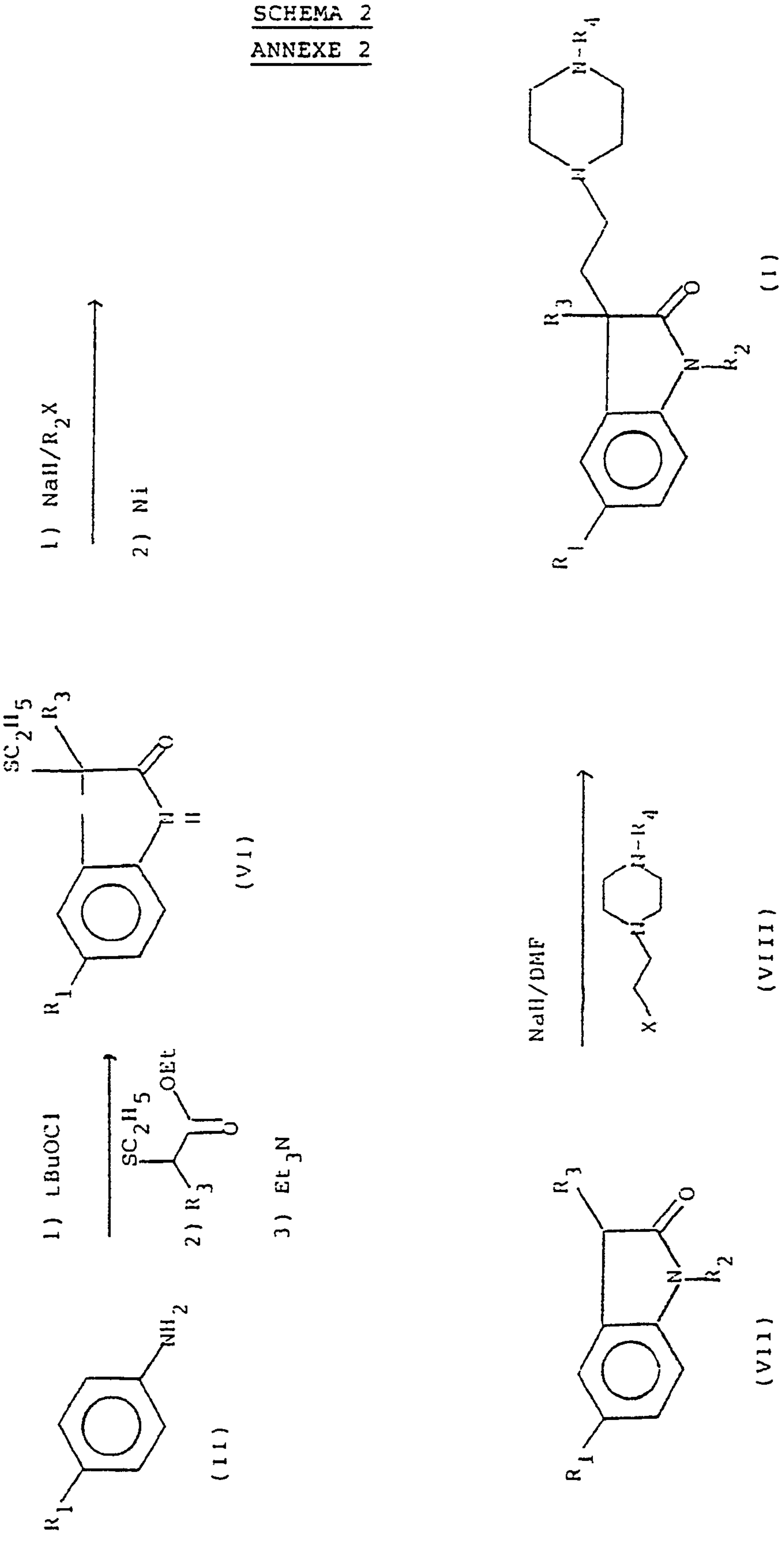


(II) (VI) (VII) (VIII) (I)

## Revendications

**Revendications pour les Etats contractants suivants : AT, BE, CH, DE, FR, GB, IT, LI, LU, NL, SE**

1. Dérivés d'indolone répondant à la formule (I)

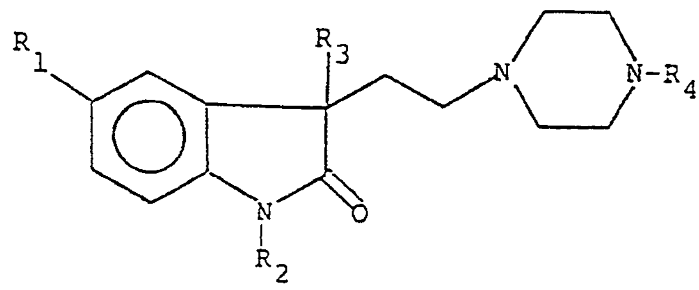

(I)

dans laquelle

— $R_1$ est un atome d'hydrogène ou d'halogène ou un radical $(C_{1-4})$alkyle,

— $R_2$ est un atome d'hydrogène ou un radical $(C_{1-4})$alkyle,

— $R_3$ est un atome d'hydrogène, un radical $(C_{1-4})$alkyle ou un radical S-$(C_{1-4})$alkyle,

— $R_4$ est un radical phényle, chloro-phényle, naphtalényle-1, méthoxy-7 naphtalényle-1, méthoxy-6 indanyle-1, méthoxy-2 pyridinyle-6, méthoxy-3 pyridinyle-2, isoquinoléinyle, méthoxy-7 isoquinoléinyle-1, méthoxy-7 tétrahydro-1,2,3,4 naphtalényle-1 ou fluoro-7 naphtalényle-1,

ainsi que les diastéréoisomères et énantiomères des composés (I) et les sels d'addition des composés (I) aux acides pharmaceutiquement acceptables.

2. La fluoro-5[[méthoxy-7 naphtalényl-1)-4 pipérazinyl-1]-2 éthyl]-3 dihydro-1,3 2H-indolone-2.

3. La fluoro-5[(méthoxy-2 pyridinyl-6)-4 pipérazinyl-1]-2 éthyl]-3 dihydro-1,3 2H-indolone-2.

4. Procédé de préparation des composés (I) dans lesquels $R_3$ est H, procédé caractérisé en ce que l'on fait réagir un composé (II), $R_1$-4 benzènamine, avec de l'hypochlorite de tertiobutyle, puis avec de l'éthylthio-3 γ-butyrolactone et enfin avec de la triéthylamine, ce qui conduit au composé (III)

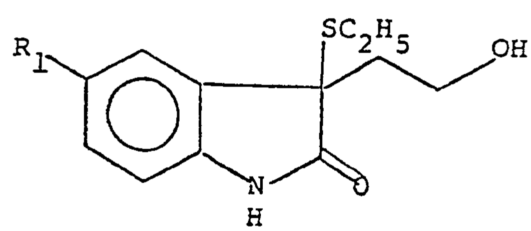

(III)

que l'on transforme en tosylate que l'on fait réagir avec une $R_4$-1 pipérazine (IV)

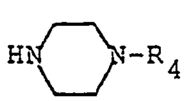

(IV)

on obtient alors un composé (V)

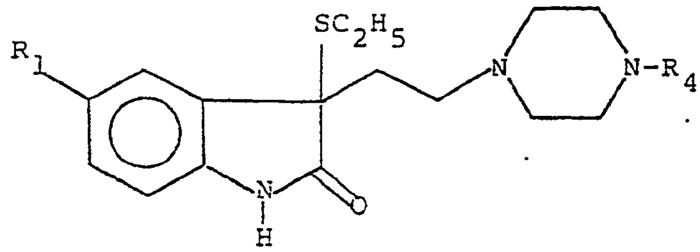

(V)

— que l'on désulfure directement pour obtenir les composés (I) dans lesquels $R_2$ est H,

— que l'on fait réagir en présence d'hydrure de sodium avec un composé $R_2X$ (dans lequel X est un atome d'halogène) puis on désulfure le composé intermédiaire, pour obtenir les composés (I) dans lesquels $R_2$ est un radical $(C_{1-4})$alkyle, les radicaux $R_1$, $R_2$ et $R_4$ ayant les significations données dans la revendication 1.

5. Procédé de préparation des composés (I) dans lesquels $R_3$ est différent de H, procédé caractérisé en ce que l'on fait réagir une ($R_1$-4) benzènamine (II) avec de l'hypochlorite de tertiobutyle, puis avec de l'éthylthio-2 $R_3$-2 éthanoate d'éthyle puis avec de la triéthylamine, ce qui conduit au composé (VI)

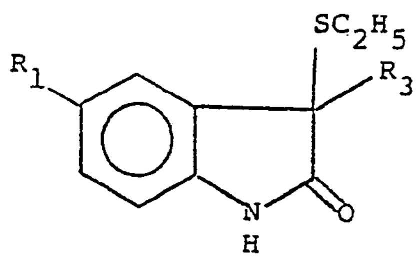

(VI)

que l'on fait réagir en présence d'hydrure de sodium avec un composé $R_2X$ puis on désulfure le composé intermédiaire en composé (VII)

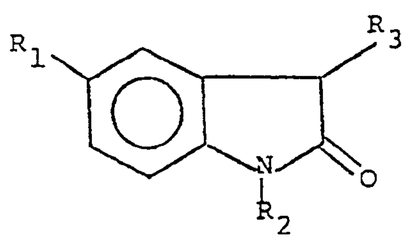

(VII)

que l'on fait réagir avec un composé (VIII)

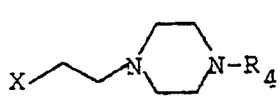

pour obtenir les composés (I),

les radicaux $R_1$, $R_2$ et $R_4$ ayant les significations données dans la revendication (I) et X est un atome d'halogène.

6. Médicament caractérisé en ce qu'il contient un composé tel que spécifié dans l'une quelconque des revendications 1 à 3.

7. Composition pharmaceutique caractérisée en ce qu'elle contient un composé tel que spécifié dans l'une quelconque des revendications 1 à 3 en association avec tout excipient approprié.

**Revendications pour les Etats contractants suivants : ES, GR**

1. Procédé de préparation de dérivés d'indolone répondant à la formule (I)

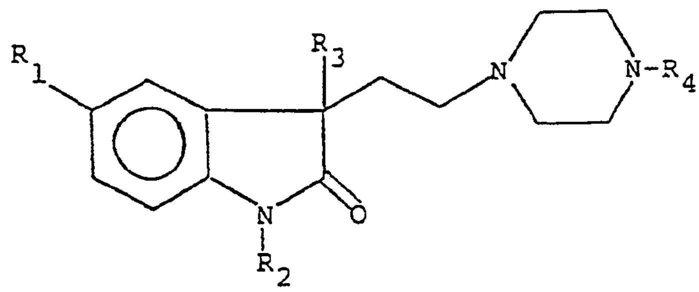

(I)

dans laquelle

— $R_1$ est un atome d'hydrogène ou d'halogène ou un radical $(C_{1-4})$alkyle,
— $R_2$ est un atome d'hydrogène ou un radical $(C_{1-4})$alkyle,
— $R_3$ est un atome d'hydrogène, un radical $(C_{1-4})$alkyle ou un radical S-$(C_{1-4})$alkyle,
— $R_4$ est un radical phényle, chloro-phényle, naphtalényle-1, méthoxy-7 naphtalényle-1, méthoxy-6 indanyle-1, méthoxy-2 pyridinyle-6 méthoxy-3 pyridinyle-2, isoquinoléinyle, méthoxy-7 isoquinoléinyle-1, méthoxy-7 tétrahydro-1,2,3,4 naphtalényle-1 ou fluoro-7 naphtalényle-1,

ainsi que les diastéréoisomères et énantiomères des composés (I) et des sels d'addition des composés (I) aux acides pharmaceutiquement acceptables,

• procédé caractérisé en ce que l'on prépare les composés dans lesquels $R_3$ est H, en faisant réagir un composé (II), $R_1$-4 benzènamine, avec de l'hypochlorite de tertiobutyle, puis avec de l'éthylthio-3 γ-butyrolactone et enfin avec de la triéthylamine, ce qui conduit au composé (III)

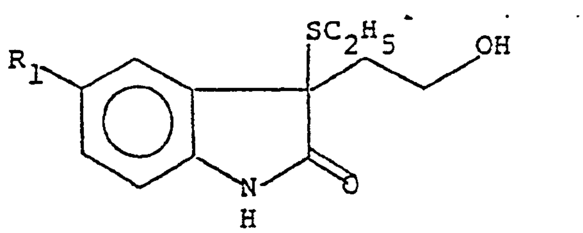

(III)

que l'on transforme en tosylate que l'on fait réagir avec une $R_4$-1 pipérazine (IV)

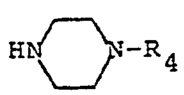 (IV),

on obtient alors un composé (V)

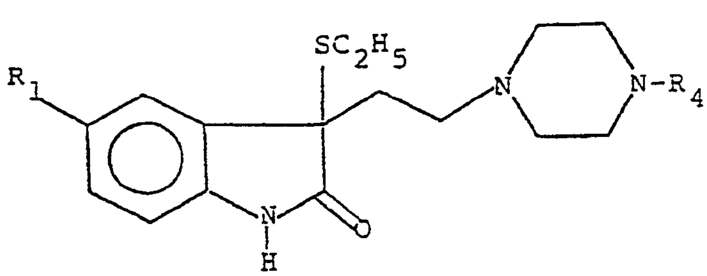

(V)

— que l'on désulfure directement pour obtenir les composés (I) dans lesquels $R_2$ est H,

— que l'on fait réagir en présence d'hydrure de sodium avec un composé $R_2X$ (dans lequel X est un atome d'halogène) puis on désulfure le composé intermédiaire, pour obtenir les composés (I) dans lesquels $R_2$ est un radical $(C_{1-4})$alkyle,

• et procédé caractérisé en ce que l'on prépare les composés dans lesquels $R_3$ est différent de H, en faisant réagir une ($R_1$-4) benzènamine (II) avec de l'hypochlorite de tertiobutyle, puis avec de l'éthylthio-2 $R_3$-2 éthanoate d'éthyle puis avec de la triéthylamine, ce qui conduit au composé (VI)

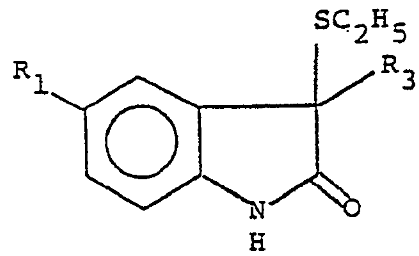

(VI)

que l'on fait réagir en présence d'hydrure de sodium avec un composé $R_2X$ puis on désulfure le composé intermédiaire en composé (VII)

(VII)

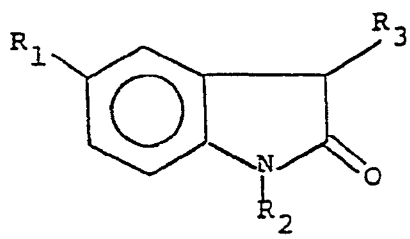

que l'on fait réagir avec un composé (VIII)

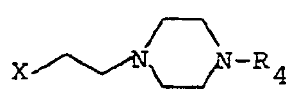

pour obtenir les composés (I).

2. Procédé selon la revendication 1, caractérisé par le fait que l'on prépare la fluoro-5[[méthoxy-7 naphtalényl-1)-4 pipérazinyl-1]-2 éthyl]-3 dihydro-1,3 2H-indolone-2.

3. Procédé selon la revendication 1, caractérisé par le fait que l'on prépare la fluoro-5[(méthoxy-2 pyridinyl-6)-4 pipérazinyl-1]-2 éthyl]-3 dihydro-1,3 2H-indolone-2.

## Patentansprüche

## Patentansprüche für folgende Vertragsstaaten : AT, BE, CH, DE, FR, GB, IT, LI, LU, NL, SE

1. Indolonderivate der Formel (I)

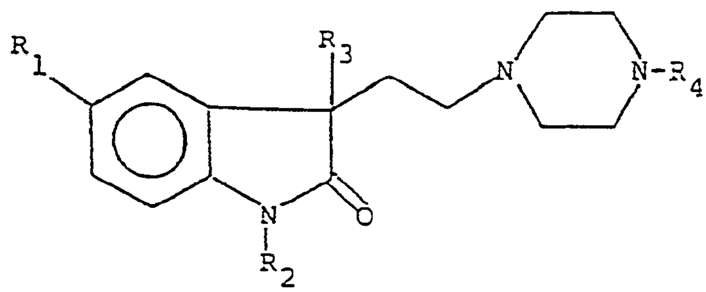

(I)

in welcher

— $R_1$ für ein Wasserstoff- oder Halogenatom oder einen $(C_{1-4})$-Alkylrest,

— $R_2$ für ein Wasserstoffatom oder einen $(C_{1-4})$-Alkylrest,

— $R_3$ für ein Wasserstoffatom, einen $(C_{1-4})$-Alkyl- oder einen S-$(C_{1-4})$-Alkylrest steht und

— $R_4$ einen Phenyl-, Chlorphenyl-, Naphthalinyl-1-, 7-Methoxynaphthalinyl-1-, 6-Methoxyindanyl-1-, 2-Methoxypyridinyl-6-, 3-Methoxypyridinyl-2-, Isochinolinyl-, 7-Methoxyisochinolinyl-I-, 7-Methoxy-1,2,3,4-tetrahydronaphthalinyl-1- oder 7-Fluornaphthalinyl-1-Rest bedeutet,

sowie die Diastereoisomeren und Enantiomeren der Verbindungen (I) und die Additionssalze der Verbindungen (I) mit pharmazeutisch verwendbaren Säuren.

2. 5-Fluor-3-[2-[4-(7-methoxynaphthalinyl-1)-piperazinyl-1]-ethyl]-1,3-dihydro-2H-indolon-2.

3. 5-Fluor-3-[2-[4-(2-methoxypyridinyl-6)-piperazinyl-1]-ethyl]-1,3-dihydro-2H-indolon-2.

4. Verfahren zur Herstellung von Verbindungen (I), in welchen $R_3$ für Wasserstoff steht, dadurch gekennzeichnet, daß man eine Verbindung (II), (4-$R_1$)-Aminobenzol, mit Tertiobutylhypochlorit, dann mit 3-Ethylthio-gamma-butyrolacton und schließlich mit Triethylamin reagieren läßt, was zur Verbindung (III)

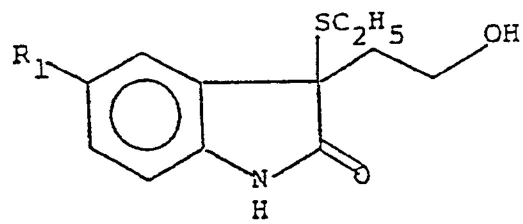

(III)

führt, die man in das Tosylat umwandelt, welches man mit einem 1-$R_4$-Piperazin (IV)

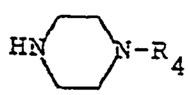

(IV)

umsetzt, worauf man eine Verbindung (V)

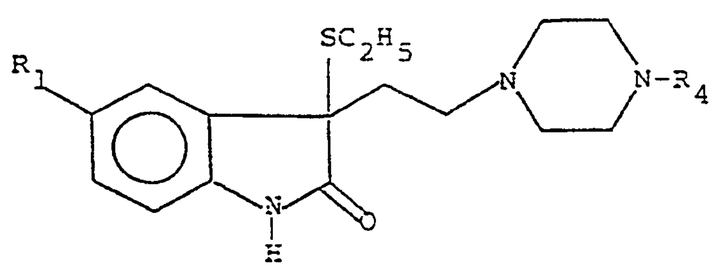

(V)

erhält, die man

— direkt entschwefelt, um die Verbindungen (I), in welchen $R_2$ für H steht, zu erhalten,

— in Gegenwart von Natriumhydrid mit einer Verbindung $R_2X$ (in welcher X für ein Halogenatom steht) umsetzt, dann die Zwischenverbindung entschwefelt, um die Verbindungen (I) zu erhalten, in welchen $R_2$ für einen ($C_{1-4}$)-Alkylrest steht, wobei die Reste $R_1$, $R_2$ und $R_4$ die in Anspruch 1 angegebene Bedeutung haben.

5. Verfahren zur Herstellung von Verbindungen (I), in welchen $R_3$ von H verschieden ist, dadurch gekennzeichnet, daß man ein (4-$R_1$)-Aminobenzol (II) mit Tertiobutylhypochlorit, dann mit Ethyl-(2-Ethylthio-2-$R_3$-ethanoat) und dann mit Triethylamin umsetzt, was zu der Verbindung (VI) führt

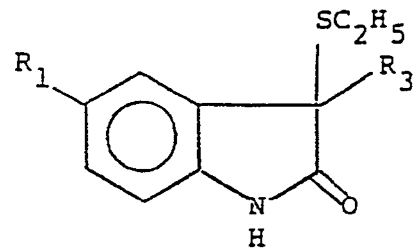

(VI)

die man in Gegenwart von Natriumhydrid mit einer Verbindung $R_2X$ umsetzt, anschließend die Zwischenverbindung zur Verbindung (VII)

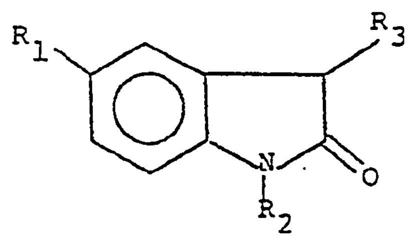

(VII)

entschwefelt, die man ihrerseits mit einer Verbindung (VIII)

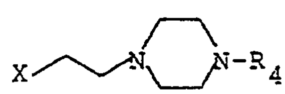

zur Reaktion bringt, um die Verbindungen (I) zu erhalten, wobei die Reste $R_1$, $R_2$ und $R_4$ die in Anspruch 1 angegebene Bedeutung haben und X für ein Halogenatom steht.

6. Arzneimittel, dadurch gekennzeichnet, daß es eine Verbindung nach einem der Ansprüche 1 bis 3 enthält.

7. Pharmazeutische Zusammensetzung, dadurch gekennzeichnet, daß sie eine Verbindung nach einem der Ansprüche 1 bis 3 in Kombination mit irgendeinem geeigneten Trägermaterial enthält.

**Patentansprüche für folgende Vertragsstaaten : ES, GR**

1. Verfahren zur Herstellung von Indolonderivaten der Formel (I)

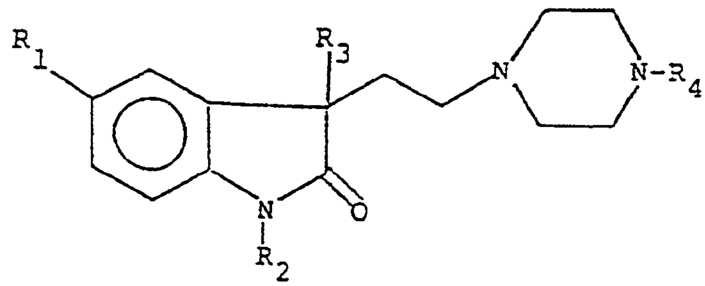

(I)

in welcher

— $R_1$ für ein Wasserstoff- oder Halogenatom oder einen $(C_{1-4})$-Alkylrest,

— $R_2$ für ein Wasserstoffatom oder einen $(C_{1-4})$-Alkylrest,

— $R_3$ für ein Wasserstoffatom, einen $(C_{1-4})$-Alkyl- oder einen S-$(C_{1-4})$-Alkylrest steht und

— $R_4$ einen Phenyl-, Chlorphenyl-, Naphthalinyl-1-, 7-Methoxynaphthalinyl-1-, 6-Methoxyindanyl-1-, 2-Methoxypyridinyl-6-, 3-Methoxypyridinyl-2-, Isochinolinyl-, 7-Methoxyisochinolinyl-1-, 7-Methoxy-1,2,3,4-tetrahydronaphthalinyl-1- oder 7-Fluornaphthalinyl-1-Rest bedeutet, sowie den Diastereoisomeren und Enantiomeren der Verbindungen (I) und den Additionssalzen der Verbindungen (I) mit pharmazeutisch verwendbaren Säuren,

• dadurch gekennzeichnet, daß man zur Herstellung der Verbindungen, in welchen $R_3$ für H steht, eine Verbindung (II), (4-$R_1$)-Aminobenzol, mit Tertiobutylhypochlorit, dann mit 3-Ethylthio-gamma-butyrolacton und schließlich mit Triethylamin reagieren läßt, was zur Verbindung (III)

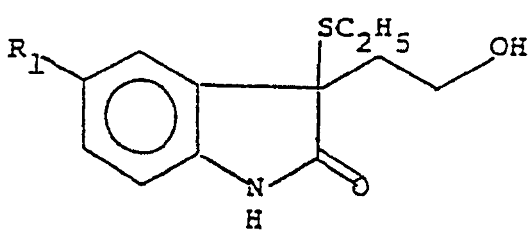

(III)

führt, die man in das Tosylat umwandelt, welches man mit einem 1-$R_4$-Piperazin (IV)

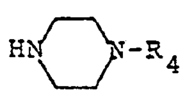

(IV),

umsetzt, worauf man eine Verbindung (V)

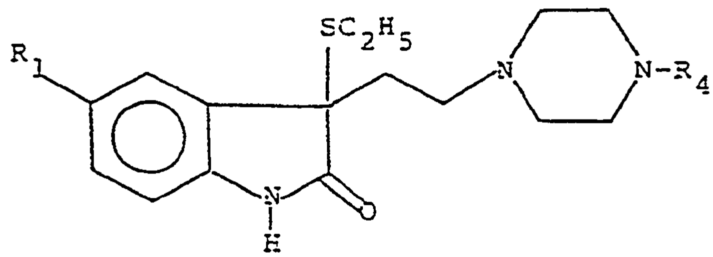

(V)

erhält, die man

— direkt entschwefelt, um die Verbindungen (I), in welchen $R_2$ für H steht, zu erhalten,

— in Gegenwart von Natriumhydrid mit einer Verbindung $R_2X$ (in welcher X für ein Halogenatom steht) umsetzt, dann die Zwischenverbindung entschwefelt, um die Verbindungen (I) zu erhalten, in welchen $R_2$ für einen $(C_{1-4})$-Alkylrest steht,

• und dadurch gekennzeichnet, daß man zur Herstellung der Verbindungen, in welchen $R_3$ von H verschieden ist, ein (4-$R_1$)-Aminobenzol (II) mit Tertiobutylhypochlorit, dann mit Ethyl-(2-Ethylthio-2-$R_3$-ethanoat) und dann mit Triethylamin umsetzt, was zu der Verbindung (VI) führt

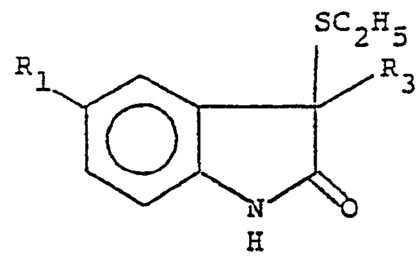

(VI)

die man in Gegenwart von Natriumhydrid mit einer Verbindung $R_2X$ umsetzt, anschließend die Zwischenverbindung zur Verbindung (VII)

(VII)

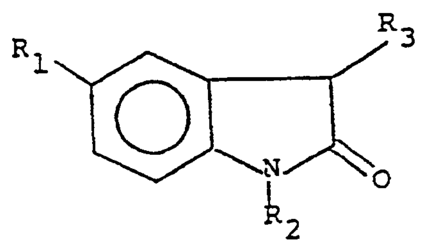

entschwefelt, die man ihrerseits mit einer Verbindung (VIII)

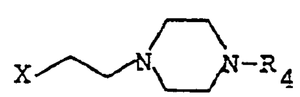

zur Reaktion bringt, um die Verbindungen (I) zu erhalten.

2. Verfahren nach Anspruch 1, dadurch gekennzeichnet, daß man 5-Fluor-3-[2-[4-(7-methoxynaphthalinyl-1)-piperazinyl-1]-ethyl]-1,3-dihydro-2H-indolon-2 herstellt.

3. Verfahren nach Anspruch 1, dadurch gekennzeichnet, daß man 5-Fluor-3-[2-[4-(2-methoxypyridinyl-6)-piperazinyl-1]-ethyl]-1,3-dihydro-2H-indolon-2 herstellt.

## Claims

### Claims for following Contracting States : AT, BE, CH, FR, GB, IT, LI, LU, NL, SE

1. Indolone derivatives corresponding to the formula (I)

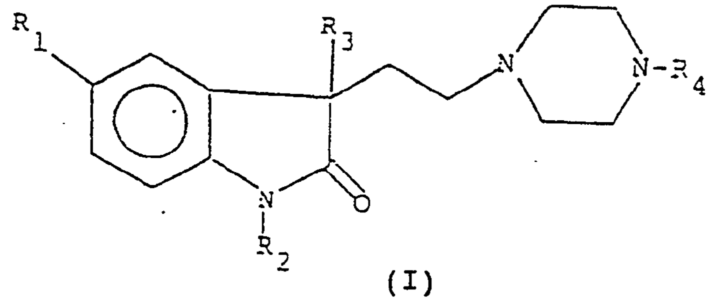

(I)

in which

— $R_1$ is a hydrogen or halogen atom or a $(C_{1-4})$ alkyl radical,

— $R_2$ is a hydrogen atom or a $(C_{1-4})$alkyl radical,

— $R_3$ is a hydrogen atom, a $(C_{1-4})$alkyl radical or an S-$(C_{1-4})$alkyl radical,

— $R_4$ is a phenyl, chlorophenyl, 1-naphthyl, 7-methoxy-1-naphthyl, 6-methoxy-1-indanyl, 2-methoxy-6-pyridyl, 3-methoxy-2-pyridyl, isoquinolyl, 7-methoxy-1-isoquinolyl, 7-methoxy-1,2,3,4-tetrahydro-1-naphthyl or 7-fluoro-1-naphthyl radical,

as well as the diastereoisomers and enantiomers of the compounds (I) and the addition salts of the compounds (I) with pharmaceutically acceptable acids.

2. 5-Fluoro-3-{2-[4-(7-methoxy-1-naphthyl)-1-piperazinyl]ethyl}-1,3-dihydro-2(2H)-indolone.

3. 5-Fluoro-3-{2-[4-(2-methoxy-6-pyridyl)-1-piperazinyl]ethyl}-1,3-dihydro-2(2H)-indolone.

4. Process for preparing the compounds (I) in which $R_3$ is H, which process is characterized in that a compound (II), 4-$R_1$-benzenamine, is reacted with tert-butyl hypochlorite, then with 3-ethylthio-γ-butyrolactone and finally with triethylamine, leading to the compound (III)

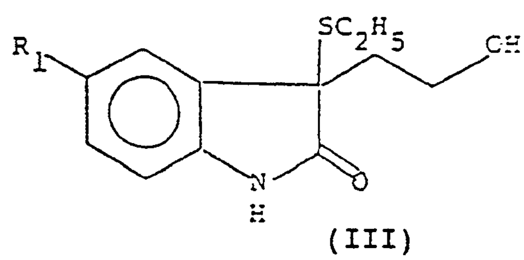

(III)

which is converted to a tosylate which is reacted with a 1-$R_4$-piperazine (IV)

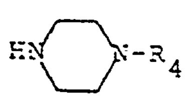

(IV);

a compound (V)

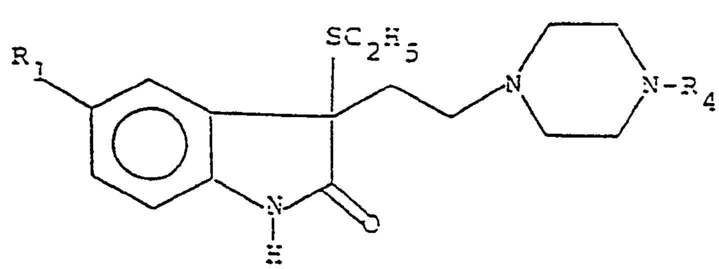

(V)

is then obtained

— which compound is desulphurized directly to obtain the compounds (I) in which $R_2$ is H,

— which compound is reacted in the presence of sodium hydride with a compound $R_2X$ (in which X is a halogen atom), and the intermediate compound is then desulphurized to obtain the compounds (I) in which

$R_2$ is a $(C_{1-4})$alkyl radical, the radicals $R_1$, $R_2$ and $R_4$ having the meanings given in Claim 1.

5. Process for preparing the compounds (I) in which $R_3$ is other than H, which process is characterized in that a 4-$R_1$-benzenamine (II) is reacted with tert-butyl hypochlorite, then with ethyl 2-ethylthio-2-$R_3$-ethanoate and then with triethylamine, leading to the compound (VI)

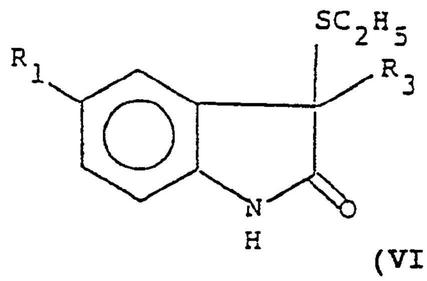

(VI)

which is reacted in the presence of sodium hydride with a compound $R_2X$, and the intermediate compound is then desulphurized to the compound (VII)

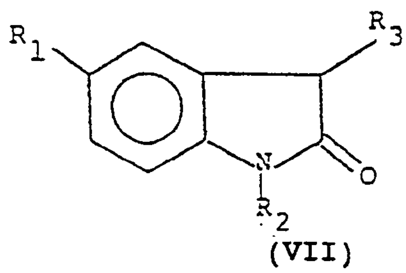

(VII)

which is reacted with a compound (VIII)

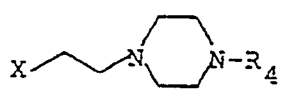

to obtain the compounds (I), the radicals $R_1$, $R_2$ and $R_4$ having the meanings given in Claim 1 and X is a halogen atom.

6. Medicinal product, characterized in that it contains a compound as specified in any one of Claims 1 to 3.

7. Pharmaceutical composition, characterized in that it contains a compound as specified in any one of Claims 1 to 3, in combination with any suitable excipient.

**Claims for the following Contracting States : ES, GR**

1. Process for preparing indolone derivatives corresponding to the formula (I)

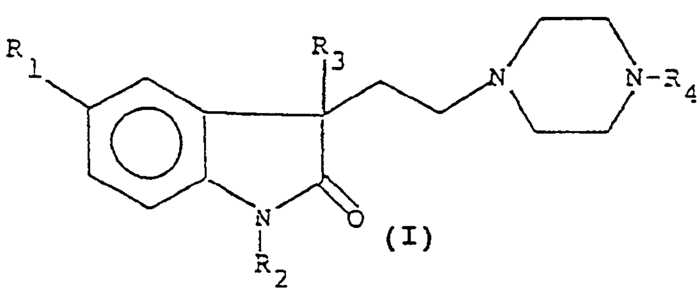

(I)

in which

— $R_1$ is a hydrogen or halogen atom or a $(C_{1-4})$ alkyl radical,

— $R_2$ is a hydrogen atom or a $(C_{1-4})$alkyl radical,
— $R_3$ is a hydrogen atom, a $(C_{1-4})$alkyl radical or an S-$(C_{1-4})$alkyl radical,
— $R_4$ is a phenyl, chlorophenyl, 1-naphthyl, 7-methoxy-1-naphthyl, 6-methoxy-1-indanyl, 2-methoxy-6-pyridyl, 3-methoxy-2-pyridyl, isoquinolyl, 2-methoxy-1-isoquinolyl, 7-methoxy-1,2,3,4-tetrahydro-1-naphthyl or 7-fluoro-1-naphthyl radical,

as well as the diastereoisomers and enantiomers of the compounds (I) and the addition salts of the compounds (I) with pharmaceutically acceptable acids,

• which process is characterized in that the compounds in which $R_3$ is H are prepared by reacting a compound (II), 4-$R_1$-benzenamine, with tert-butyl hypochlorite, then with 3-ethylthio-γ-butyrolactone and finally with triethylamine, leading to the compound (III)

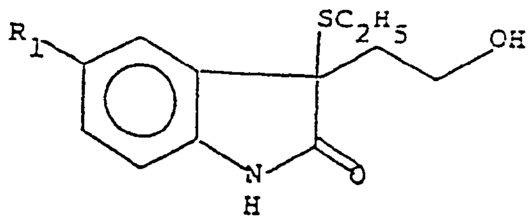

(III)

which is converted to a tosylate which is reacted with a 1-$R_4$-piperazine (IV)

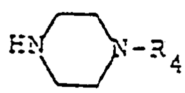

(IV);

a compound (V)

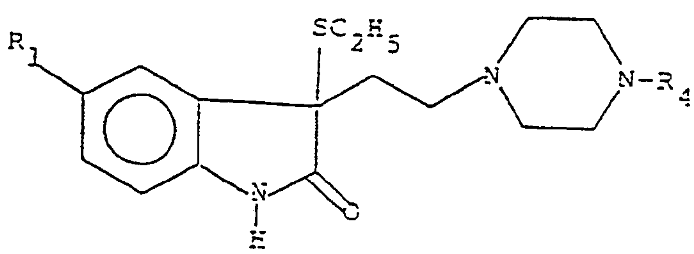

(V)

is then obtained

— which compound is desulphurized directly to obtain the compounds (I) in which $R_2$ is H,
— which compound is reacted in the presence of sodium hydride with a compound $R_2X$ (in which X is a halogen atom), and the intermediate compound is then desulphurized to obtain the compounds (I) in which $R_2$ is a $(C_{1-4})$alkyl radical,

• and which process is characterized in that the compounds in which $R_3$ is other than H are prepared by reacting a 4-$R_1$-benzenamine (II) with tert-butyl hypochlorite, then with ethyl 2-ethylthio-2-$R_3$-ethanoate and then with triethylamine, leading to the compound (VI)

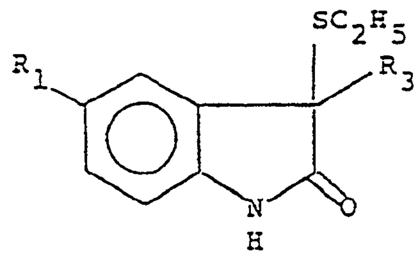

(VI)

which is reacted in the presence of sodium hydride with a compound $R_2X$, and the intermediate compound is then desulphurized to the compound (VII)

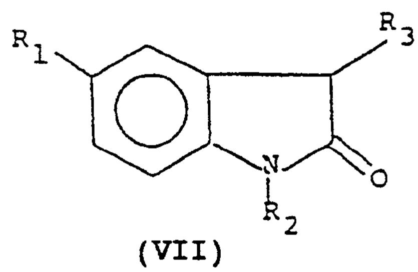

(VII)

which is reacted with a compound (VIII)

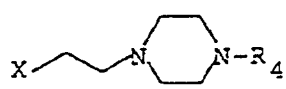

to obtain the compounds (I).

2. Process according to Claim 1, characterized in that 5-fluoro-3-{2-[4-(7-methoxy-1-naphthyl)-1-piperazinyl]ethyl}-1,3-dihydro-2(2H)-indolone is prepared.

3. Process according to Claim 1, characterized in that 5-fluoro-3-{2-[4-(2-methoxy-6-pyridyl)-1-piperazinyl]ethyl}-1,3-dihydro-2(2H)-indolone is prepared.